# EUROPEAN PATENT SPECIFICATION

(11) **EP 2 310 051 B1**
(45) Date of publication and mention of the grant of the patent: **25.02.2015**
(21) Application number: 09772865.3
(22) Date of filing: 01.07.2009
(51) Int. Cl.: A61K 31/05, A61K 31/565, A61K 31/65, A61K 45/06

(54) **COMBINATION OF A PARP INHIBITOR AND AN AKT KINASE ACTIVATING COMPOUND**
KOMBINATION EINES PARP-HEMMERS UND EINER AKT-KINASE AKTIVIERENDEN SUBSTANZ
COMBINAISON D'UN INHIBITEUR DE PARP ET D'UNE SUBSTANCE ACTIVANT LA KINASE AKT

(30) Priority: 04.07.2008 HU 0800414
(43) Date of publication of application: 20.04.2011
(73) Proprietor: Pécsi Tudomànyegyetem, 7633 Pécs (HU)
(72) Inventor: GALLYAS, Ferenc, H-7633 Pécs (HU); SÜMEGI, Balázs, H-7634 Pécs (HU); VETÖ, Sára, H-7627 Pécs (HU); ÁCS, Péter, H-7625 Pécs (HU); KOMOLY, Sámuel, H-1146 Budapest (HU); ILLÉS, Zsolt, H-7635 Pécs (HU)
(74) Representative: Török, Ferenc
(86) International application number: PCT/HU2009/000055
(87) International publication number: WO 2010/001186

(56) References cited:
- WO-A1-02/20022
- WO-A1-99/08680
- WO-A2-2007/038636
- WO-A2-2008/013764
- US-A1- 2004 092 444
- BECKERT STEFAN ET AL: "IGF-I-induced VEGF expression in HUVEC involves phosphorylation and inhibition of poly(ADP-ribose)polymerase" BIOCHEMICAL AND BIOPHYSICAL RESEARCH COMMUNICATIONS, vol. 341, no. 1, March 2006 (2006-03), pages 67-72, XP005253032 ISSN: 0006-291X
- SHANG YOU ET AL: "Protective effect of erythropoietin against ketamine-induced apoptosis in cultured rat cortical neurons: Involvement of PI3K/Akt and GSK-3 beta pathway" APOPTOSIS, vol. 12, no. 12, December 2007 (2007-12), pages 2187-2195, XP019548133 ISSN: 1360-8185
- ALANO CONRAD C ET AL: "Minocycline inhibits poly(ADP-ribose) polymerase-1 at nanomolar concentrations" PROCEEDINGS OF THE NATIONAL ACADEMY OF SCIENCES OF THE UNITED STATES OF AMERICA, vol. 103, no. 25, June 2006 (2006-06), pages 9685-9690, XP002554684 ISSN: 0027-8424
- CIMAROSTI HELENA ET AL: "Estradiol protects against oxygen and glucose deprivation in rat hippocampal organotypic cultures and activates Akt and inactivates GSK-3 beta" NEUROCHEMICAL RESEARCH, vol. 30, no. 2, February 2005 (2005-02), pages 191-199, XP019289802 ISSN: 0364-3190

## Description

### Field of the invention

The subject of the invention is a combined use and the relating pharmaceutical composition wherein the combination of a PARP inhibitor and an Akt kinase activating compound is applied as active ingredient. The invention is useful in the treatment of neurodegenerative diseases where PARP inhibition and/or activation of one of the most important cytoprotective protein kinase, Akt, is advantageous in curing the disease.

### Background of the invention

In the past few years it has been discovered in many diseases that somehow they are connected to increased PARP activation. Ischaemic reperfusion heart injury [Methods in Enzymol. 186: 1-85, 1990; Arch. Biochem. Biophys. 288:533-7, 1991], oxidative damage in different cells [Adv. Exp. Med. Biol. 361:319-325, 1994; Mol. Cell. Biochem. 138: 141-8, 1994], neuronal ischaemia [Nat. Med. 3, 1089-1095, 1997; J. Cereb. Blood Flow Metab. 17, 1143-1151, 1997], acute pneumonia [Am. J. Respir. Crit. Care Med. 165, 372-377, 2002], acute septic shock [Shock 17, 286-292, 2002], zymogen induced multiple organ failure [J. Exp. Med. 186, 1041-1049, 1997] and diabetic pancreas damage, as well as illness associated with these pathologies typically fall into this category. According to the latest results, PARP enzyme plays an important role in the pathomechanism of numerous central nervous system and neurodegenerative disorders [J. Pharmacol. Exp. Ther., 310(3):1053-61, 2004; J. Neurosci. Res., 81(2):190-8, 2005; Curr. Pharm. Des. 13(8): 933-62, 2007]. In case of neurodegenerative diseases (for example multiple sclerosis, encephalomyelitis, Parkinson's, Alzheimer's, Huntington's disease and other degenerative disorders), available treatments offer relatively small symptomatic benefits and remain palliative in nature. These diseases affect a large number of people, for example multiple sclerosis (MS) is one of the most common neurological diseases among European and North American young population. This chronic inflammatory central nervous system (CNS) disease effects around 1 million people worldwide. Active MS histological changes include infiltration of the CNS by T and B lymphocytes and macrophages, myelin degradation andoligodendrocyte degeneration (demyelination) followed by partial reconstruction (remyelination), axon lesions (neurodegradation), and astrocyte and microglia activation. According to the common view, these inflammatory changes imply autoimmune attacks on myelin components. Accordingly, experimental autoimmune encephalomyelitis (EAE) is one of the most accepted animal models of the disease.

An alternative hypothesis to the heterogeneous pathogenesis of MS proposed that oligodendrocyte apoptosis represents the first and earliest stage of all lesions. According to that model, autoimmune inflammation may be secondary due to apoptosis of oligodendrocyts resulting in primary demyelination unmasking tissue antigens [Barnett, M.H. & Prineas, J.W. Relapsing and remitting multiple sclerosis: pathology of the newly forming lesion. Ann. Neurol. 55, 458-468 (2004)]. Besides loss of oligodendrocyts in the earliest lesions, depletion of oligodendrocyts progressively occurs during lesion evolution [Frohman, E.M., Racke, M.K. & Raine, C.S. Multiple sclerosis - the plaque and its pathogenesis, N. Engl. J. Med. 354, 942-955 (2006)]. This patomechanism applies especially to MS type 3 and 4 and, but can explain the pathogenesis of other types of MS as well.. Cuprizone induced demyelization is suggested for modeling such degenerative MS types. This model is commonly used for studying de- and remyelization occurring during MS. Cuprizone [bis(cyclohexanone) oxaldihydrazone] forms a chelate complex with Cu⁺⁺ ions and induces demyelination primarily in the corpus callosum and superior cerebral pedunculum. Termination of the treatment results in rapid remyelination.

Based on our own studies and published evidences *(*Hemm RD, Carlton WW, Welser JR: Ultrastructural changes of cuprizone encephalopathy in mice, Toxicol. Appl. Pharmacol. 1971 Apr; 18(4):869-82*.;* Kesterson JW, Carlton WW.: Aqueductal stenosis as the cause of hydrocephalus in mice fed the substituted hydrazine, cuprizon, Exp. Mol. Pathol. 1970 Dec.;13(3):281-94*.),* cuprizone also induces formation of hydrocephalus, thus the cuprizone model can also be used in disorders leading to hydrocephalus. Accordingly, during our experiments, we examined both demyelination and the formation of hydrocephalus.

Parallel to the accumulation of theoretical material, numerous immunmodulation therapies were experimented, and new agents (IFN-ß1a, IFN-ß1b, glatiramer-acetate, mitoxantrone) were introduced into the clinical management of MS. Despite of the fact that the new therapies drastically changed the MS treatment, there is no such current therapy which is able to stop the course of the disease, so active research is still being done to find more effective treatment.

Poly ADP ribose polymerase (PARP), a nuclear enzyme occuring in a highcopy number is activated by oxidative stress-induced DNA-breaks. Upon activation, PARP cleaves nicotinamide group from NAD⁺ and ADP-ribosylates nuclear proteins. PARP activation affects numerous nuclear processes such as DNA repair and control of transcription. Among others, expression of NFκB-regulated genes during inflammatory processes needs PARP activation, which is proven by the fact that expression of these genes is greatly damaged in PARP-1^{-/-} mice (mice missing PARP-1). PARP1^{-/-} mice proved resistant to inflammatory processes and septic shock while no serious changes were found in these animals.

According to our pervious results, PARP inhibitors [4-hydroxy-quinazoline (4-HQ), PJ-34 (N-(6-oxo-5,6-dihydrophenanthridin-2-yl)-N,N-dimethylacetamide.HCl)] were successful in preventing LPS induced septic shock in normal BALB/c mice and were effective against various oxidative stress stimuli [Biochem Pharmacol. 65(8) 1373-1382, 2003; J. Biol. Chem. 280(42) 35767-75, 2005]. Our research group was the first to document that in the cytoprotective effect of PARP inhibitors the activation of PI3 kinase/Akt pathway plays a significant role during septic shock. Results of other research groups showed that PJ-34 effectively decreased MBP induced formation of EAE in PJSJL mice [Pharmacol. Res. 52(1):109-18, 2005], and two other articles also demonstrated the role of PARP in neuroinflammatory processes [J. Exp. Med. 198(11):1729-40, 2003; J. Neurochem. 85(2):306-17,2003].

It is well known that Akt kinase (Akt) activation protects cells from stress under different conditions, and literature clearly shows that Akt is a very important cytoprotective kinase (Chong ZZ, Kang JQ, Maiese K.: Essential cellular regulatory elements of oxidative stress in early and late phases of apoptosis in the central nervous system, Antioxid. Redox Signal. 2004 Apr;6(2):277-87*.;* Nair VD, Olanow CW.: Differential Modulation of Akt/Glycogen Synthase Kinase-3{beta} Pathway Regulates Apoptotic and Cytoprotective Signaling Responses, J. Biol. Chem. 2008 May 30; 283(22):15469-78*.,* Quesada A, Lee BY, Micevych P: PI3 kinase/Akt activation mediates estrogen and IGF-1 nigral DA neuronal neuroprotection against a unilateral rat model of Parkinson's disease. Dev Neurobiol. 2008 Apr; 68(5):632-44*.,* Zhou P, Qian L, Chou T, Iadecola C.: Neuroprotection by PGE2 receptor EP1 inhibition involves the PTEN/AKT pathway, Neurobiol. Dis: 2008 Mar; 29(3):543-51) This protection is exerted partially by protecting the mitochondirum, namely via phosphorylation and therby inactivation of the proapoptotic Bad (Mullonkal CJ, Toledo-Pereyra L: Akt in ischemia and reperfusion, J. Invest. Surg. 2007 May-Jun;20(3):195-203). Because one of the early effects of cuprizone is the formation of megamitochondria, it was proposed that via protection of mitochondria, we can protect the degenerating cells. Thus we looked for non-toxic materials which activate Akt and thus has the potential to help the regeneration of the damaged cells in the nervous system.

Furthermore its known that estrogens also have a neuroprotective effect, which makes them useful in the treatment of neurodegenerative diseases (see estrogen compound family disclosed in EP 1 511 496 B1). This effect can be traced back to the PARP inhibitory effect of estrogen which may be exerted in a way where the estrogen binds itself to alpha receptor and forms a complex with PARP, i.e. anchors PARP to the DNA and thus prevents DNA break-induced activation of PARP [J. Pharmacol. Exp. Ther. 315(2):812-20, 2005].

In recent years, it was also discovered that stilbene compounds, for example trans-resveratrol, activate Akt *(*Das S, Fraga CG, Das DK.: Cardioprotective effect of resveratrol via HO-1 expression involves p38 map kinase and PI-3-kinase signaling, but does not involve NFkappa, Free Radic. Res. 2006 Oct.; 40(10):1066-75*.;* Das S, Tosaki A, Bagchi D, Maulik N, Das DK: Resveratrol-mediated activation of cAMP response element-binding protein through adenosine A3 receptor by Akt-dependent and -independent pathways, J. Pharmacol. Exp. Ther. 2005 Aug.; 314(2):762-9*.).* This was the basis of the idea that these compounds combined with PARP inhibitors could be beneficial in neurodegenerative and demyelinating diseases.

Recent discoveries showed that substances with tetracycline structure also exhibit PARP inhibitory effects. For example, minocycline inhibits PARP activity in a concentration of 1 nanomol [Proc. Natl. Acad. Sci. USA, 103(25):9685-90, 2006]. Similar findings were made in the following tetracycline compounds: doxycycline, demeclocycline, clortetracycline.

It is also known that doxycycline in combination with an interferon compound (interferon beta 1a) showed positive effect in the treatment of relapsing remitting sclerosis multiplex (RRMS) (on-line publication, Archives of Neurology, December 10, 2007).

However neither of the above mentioned pathways proved useful in the treatment of neurodegenerative diseases.

Oxidative stress plays an important role in many pathological processes. Here we can mention cardio and cerebrovascular clinical profiles belonging to ischemia reperfusion diseases, septic shock, neurodegenerative diseases, also cellular death as a consequence of anti tumor and anti viral therapy. Reactive oxygen and nitrogen species (ROS) formed in different ways induce oxidative stress and damage different intracellular componentssuch as nucleic acids, proteins and lipids. DNA break induced PARP activation is a major mechanism of ROS-induced cell death, in which p53 and NFkB mediators play an important role. Similarly important are the activation of MAP kinase pathways induced by TNF and ROS. Integrity of the mitochondrial membrane system play acentral role in regulation of signal pathways leading to cellular damage as well as mediating cell death.

However, some genes activated by PARP and MAP kinase are not known or only partially known, and they may play an important role in mediating oxidative damage. It is not known how these signaling pathways act on mitochondrial integrity, the energy producing system and mitochondrial membrane potential, and the effect of mitochondrial integrity on controlling the signaling pathways is also unknown.

### Summary of the invention

The invention is based on the recognition that use of PARP inhibitors and Akt kinase activating compounds (for example estrogens and stilbenes) in combination resulted in an unexpected protection in relevant animal models of neurodegenerative diseases. It is very surprising that the positive effects measured during the combined use are higher than the sum of the positive effects of the individual components, i.e. synergy is occurred in the combined use of the two above active agents.

### Detailed description of the invention

In the combination according to the invention the following molecules can be used as PARP inhibitors:
4-hydroxyquinazoline and its derivatives, for example compounds of Hungarian patent application published under No. P0301173, further more: Pálfi A, Tóth A, Kulcsár G, Hantó K, Deres P, Bartha E, Halmosi R, Szabados E, Czopf L, Kalai T, Hideg K, Sümegi B, Tóth K.: The role of Akt and mitogen-activated protein kinase systems in the protective effect of poly(ADP-ribose) polymerase inhibition in Langendorff perfused and in isoproterenol-damaged rat hearts, J. Pharmacol. Exp. Ther., 2005 Oct., 315(1):273-82.

Carboxamino-benzimidazole and its derivatives, for example compounds of Hungarian patent application published under No. P0400883, further more: Kovacs K, Toth A, Deres P, Kalai T, Hideg K, Gallyas F Jr, Sumegi B.: Critical role of PI3-kinase/Akt activation in the PARP inhibitor induced heart function recovery during ischemia-reperfusion, Biochem. Pharmacol., 2006 Feb 14, 71(4):441-52.

4-aminonaphtalimide and itsderivatives, for example: Sharma SS, Kumar A, Kaundal RK.: Protective effects of 4-amino-1,8-napthalimide, a poly(ADP-ribose) polymerase inhibitor in experimental diabetic neuropathy, Life Sci., 2008 Mar 12, 82(11-12):570-6.

PJ34 homologues, for example: Szijártó A, Batmunkh E, Hahn O, Mihály Z, Kreiss A, Kiss A, Lotz G, Schaff Z, Váli L, Blázovics A, Geró D, Szabó C, Kupcsulik P.: Effect of PJ-34 PARP-inhibitor on rat liver microcirculation and antioxidant status, J. Surg. Res., 2007 Sep, 142(1):72-80; Veres B, Gallyas F Jr, Varbiro G, Berente Z, Osz E, Szekeres G, Szabo C, Sumegi B.: Decrease of the inflammatory response and induction of the Akt/protein kinase B pathway by poly-(ADP-ribose) polymerase 1 inhibitor in endotoxin-induced septic shock, Biochem. Pharmacol., 2003 Apr 15, 65(8):1373-82.

Tetracyclin derivatives for example: Conrad C. Alano, Tiina M. Kauppinen, Andreu Viader Valls, and Raymond A. Swanson: Minocycline inhibits poly(ADP-ribose) polymerase-1 at nanomolar concentrations, PNAS (2006) 103, 9685-9690. Some of these compounds are medicines which are available on the market (for example doxycycline).

The Akt kinase activating compounds used in the combination according to the invention can be estrogen compounds, e.g. estrone, corticosteron, dexametaxon, estradiol, estriol and other steroids, e.g. pregnane structured compounds, preferably prednisolone.

It is know that estrogen compounds activate PI-3 kinase Akt signaling pathways *(*Marino M, Galluzzo P, Ascenzi P.: Estrogen signaling multiple pathways to impact gene transcription. Curr Genomics, 2006 Nov; 7(8): 497-508*.)* and promote cellular growth and strengthen them against different stress situations *(*Patten RD, Karas RH: Estrogen replacement and cardiomyocyte protection, Trends Cardiovasc. Med. 2006 Apr; 16(3):69-75*.).*

Thus, we assume (not binding ourselves to this theory) that estrogen type materials protect neurons in different neurological diseases.

In the combination according to the invention the Akt kinase activating compound can be a stilbene structured compound which compound family belongs to plant-originated polyphenols. Preferred are the hydrostilbene compounds, for example resveratrol. These compounds activate PI-3 kinase Akt pathway and thus most likely strengthen neuron resistance which can be achieved partially by inhibition of phsophoinositide 3 kinase phosphatase and partially via other mechanisms (Das S, Khan N, Mukherjee S, Bagchi D, Gurusamy N, Swartz H.: DK-Redox regulation of Resveratrol-mediated Switching of Death Signal into Survival Signal, Free Radic. Biol. Med., 2008 Jan 1, 44(1):82-90.). Furthermore, this effect can also be strengthened by general antioxidants, see Shankar S, Singh G, Srivastava RK.: Chemoprevention by Resveratrol: Molecular Mechanisms and Therapeutic Potential, Front Biosci., 2007 Sep 1, 12:4839-54.

Other natural stilbene structured compounds, which can be applied in the combination according to the invention, are disclosed in the following article: M.T. Ribeiro de Lima et al.: Determination of Stilbenes (trans-Astringin, cis- and trans-Piecid and cis- and trans-Resveratrol) in Portuguese Wines, J. Agric. Food Chem., 1999,47,2666-2670.

The use of stilbene structured compounds is preferable because estrogen is a female hormone thus the administration of it to men is not advisable.

In a preferred embodiment of the invention the PARP inhibitor and the Akt kinase activating compound are formulated in a common dosage unit. This dosage unit can be considered as a typical pharmaceutical composition, despite of the fact that it contains two compounds. This pharmaceutical composition of the invention can be administered in different ways, thus orally, in a liquid or in a solid form, in a solution of an injection or infusion, or even in another type of composition, e.g. as sublingual, parenteral or rectal composition.

In another preferred embodiment the different compounds are formulated into different dosage units. Preferably, these units are placed in kits, where the dosage regime can be learnt from the positioning of the dosage units, from the notes written on the kit or from the guidelines packed together with the kit. Any typical method can be used in preparing the kits, and it is not necessary that the dosage units of the different active agents are formulated in the same way (one compound can be a pill while the other can be formulated as an injection).

In each embodiment it is possible that more than one active agent is used within the same group of the active agents (i.e. more than one PARP inhibitor and/or more than one Akt kinase activating agent is applied, even formulated differently), obviously in the amount necessary for treatment of that given disease. So, in the entire specification (i.e. in the set of claims, too) the phrase "a PARP inhibitor" and "an Akt kinase activating agent" may really stand for a single active agent, but they embrace also those possibilities where more than one representative of that type of active agent is applied.

Orally administered compositions could be in the form of powder, capsule, pill, film coated pill, microcapsule etc, and they can contain carriers such e.g. gelatin, sorbitol, polyvinylpyrrolidon; filling agents such e.g. lactose, glucose, starch, calcium phosphate; tableting auxiliaries such e.g. magnesium stearate, polyethylenglycol, silica; wetting agents such e.g. sodium lauril sulfate.

Liquid compositions can be solutions, emulsions which are water based and can be also a gel.

Since the form of the dosage units (pharmaceutical compositions) according to the invention is not a characterizing feature of the invention, any known methods, techniques and materials can be used for the preparation of them. The examples of the applicable materials and methods can be found in Remington's Pharmaceutical Sciences, 18th edition, Mack Publishing Co., Easton, USA, (1990).

During the determination of the dosage of the active agents used in combination, a practitioner should start out from the normal dosage, but the general condition, age, weight of patient should be considered. Moreover, it should kept in mind that, coming from the synergic effect, smaller dosage can exert the desired effect. The applicable dosage can be determined routinely on the basis of general knowledge of the doctor.

The preferred combinations of the invention are shown in the following examples, without aiming at to limit the scope of invention to these embodiments.

### Experimental Results

Combined effect of PARP inhibitors and Akt kinase activating agents (preferably estrogen or stilbene derivatives) on cuprisone induced changes in the central nervous system of C57BL/6 mice

### General experiment description

Unless stated otherwise the following procedure was followed in examples 1-4.

### Animals

During the experiment C57BL/6 mice were kept in a standard environment, they were given tap water and ground mice feed *ad libitum.* The mice were taken care of according to ethical requirements relating to treatment of animals.

According to a previously described method [Journal of Neuroimmunology 92 (1998): 38-49], 7-8 week old C57BL/6 male mice were treated for 6 weeks *per os* with cuprizone (Sigma, Steinheim, Germany). The mice were divided into 5 groups with 8 mice in each group. The first group received no treatment, the second group was treated with cuprizone, while groups 3 to 5 were given the active agents described in examples 1 to 4 (see the active agents of examples 3 to 5).

Cuprizone was mixed at a concentration of 0.2 m/m % (% by mass) into the ground feed. Oxidation of cuprizone was prevented by giving fresh feed to the mice every day. PARP inhibitors and/or Akt kinase activating agents were placed in physiological saline solution, filtered to sterility, and administered intraperitoneally in the proper dosage. The treatment with the active agents was started a day before cuprizone was administered. Control animals were given i.p. an amount of sterile physiological solution corresponding to their bodymass.

### MRI

It is known from the literature (NMR Biomed. 2005 Oct; 18(6):395-403) that T1 and T2 MRI image intensity correlates with the degradation and regeneration of the myelin sheath in this model. Oedema and hydrocephalus registers as hyperintensity on the T2 image. For quantification of hydrocephalus MRI is more suitable than histology because during fixation and histological processing there is significant shrinkage and volume degradation (Exp. Neurology 118 (1992): 1-6). MRI micro image technique makes it possible to study the above changes *in vivo* for weeks.

After three weeks, the mice were placed into the NMR instrument in narcosis every week [5 mg/kg bodymass diazepam (Seduxen) and 90 mg/kg bodymass ketamine (Calypsol, Richter Gedeon Zrt., Budapest) intraperitoneally]. T1 and T2 images were acquired with Varian ^{UNITYI}INOVA NMR spectrometer from the bulbus olfactorius to the beginning of the spinal cord in transverse sections of 1 mm thickness. Signal intensity of a glass tube with 1 mm internal diameter, which was filled with 9:1 water-glycerin mixture, was used as outer control. The development and size of demyelinization was determined by comparing average signal intensity detected at the area of the corpus callosum with the average intensity of the outer control. Average signal intensity of the corpus callosum (CC) was determined by an expert who was blind to the experiment from the section 1 mm posterior to the bregma by manually marking the region of interest (ROI), i.e. the corpus callosum (CC). Intensity of ROI was normalized to the intensity of the control tube.

The development and the degree of hydrocephalus were quantified by calculating the chamber/brain volume ratio. The area of the chambers was outlined manually in each section, and the whole volume was calculated as the sum of the areas multiplied with the thickness of the sections (1 mm). The same method was used for the determination of the volume of chambers.

The results are given in the tables, on the one hand, as the chamber/brain volume ratio (vol. ratio) values and, on the other hand, as CC/control tube values which are the ratios of the intensities measured in corpus callosum (CC) and in the control tube.

### Example 1

### Treatment with doxycycline (PARP-1 inhibitor) (1 mg/kg bodymass) and estriol (0,4 mg/kg bodymass)

**Demyelinization after 5 weeks of treatment, determined with MRI**

| | CC/control tube |
|---|---|
| Untreated | 0,6±0,11 |
| Cuprizone treatment | 1,4±0,15 |
| Cuprizone treatment + doxycycline | 1,1±0,18 |
| Cuprizone treatment + estriol | 1,2±0,13 |
| Cuprizone treatment + estriol + doxycycline | 0,7±0,14 |

**Hydrocephalus after 5 weeks of treatment, determined with MRI**

| | Chamber/brain vol. ratio |
|---|---|
| Untreated | 0,057±0,013 |
| Cuprizone treatment | 0,192±0,017 |
| Cuprizone treatment + doxycycline | 0,128±0,012 |
| Cuprizone treatment + estriol | 0,134±0,02 |
| Cuprizone treatment + estriol + doxycycline | 0,067±0,014 |

### Example 2

### Treatment with doxycycline (PARP-1 inhibitor) (1 mg/kg bodymass) and trans-resveratrol (0,4 mg/kg bodymass)

**Demyelinization after 5 weeks of treatment, determined with MRI**

| | CC/control tube |
|---|---|
| Untreated | 0,6±0,15 |
| Cuprizone treatment | 1,5±0,12 |
| Cuprizone treatment + doxycycline | 1,2±0,17 |
| Cuprizone treatment + resveratrol | 1,3±0,09 |
| Cuprizone treatment + resveratrol + doxycycline | 0,75±0,15 |

**Hydrocephalus after 5 weeks of treatment, determined with MRI**

| | Chamber/brain vol. ratio |
|---|---|
| Untreated | 0,06±0,01 |
| Cuprizone treatment | 0,183±0,019 |
| Cuprizone treatment + doxycycline | 0,117±0,02 |
| Cuprizone treatment + resveratrol | 0,138±0,017 |
| Cuprizone treatment + resveratrol + doxycycline | 0,07±0,016 |

### Example 3

### Treatment with 4-hydroxyquinazoline (4-HQ) (PARP-1 inhibitor) (20 mg/kg bodymass) and trans-resveratrol (0,4 mg/kg bodymass)

**Demyelinization after 5 weeks of treatment, determined with MRI**

| | CC/control tube |
|---|---|
| Untreated | 0,6±0,15 |
| Cuprizone treatment | 1,5±0,12 |
| Cuprizone treatment + 4-HQ | 1,25±0,14 |
| Cuprizone treatment + resveratrol | 1,3±0,09 |
| Cuprizone treatment + resveratrol + 4-HQ | 0,7±0,11 |

**Hydrocephalus after 5 weeks of treatment, determined with MRI**

| | Chamber/brain vol. ratio |
|---|---|
| Untreated | 0,06±0,01 |
| Cuprizone treatment | 0,183±0,019 |
| Cuprizone treatment + 4-HQ | 0,122±0,03 |
| Cuprizone treatment + resveratrol | 0,138±0,017 |
| Cuprizone treatment + resveratrol + 4-HQ | 0,065±0,012 |

### Example 4

### Treatment with carboxamino-benzimidazole (Carb) (PARP-1 inhibitor) (2 mg/kg bodymass) and trans-resveratrol (0.4 mg/kg bodymass)

**Demyelinization after 5 weeks of treatment, determined with MRI**

| | CC/control tube |
|---|---|
| Untreated | 0,6±0,15 |
| Cuprizone treatment | 1,5±0,12 |
| Cuprizone treatment + Carb | 1,24±0,08 |
| Cuprizone treatment + resveratrol | 1,3±0,09 |
| Cuprizone treatment + resveratrol + Carb | 0,72±0,13 |

**Hydrocephalus after 5 weeks of treatment, determined with MRI**

| | Chamber/brain vol. ratio |
|---|---|
| Untreated | 0,06±0,01 |
| Cuprizone treatment | 0,183±0,019 |
| Cuprizone treatment + Carb | 0,118±0,05 |
| Cuprizone treatment + resveratrol | 0,138±0,017 |
| Cuprizone treatment + resveratrol + Carb | 0,063±0,010 |

The results indicated synergy between the two agents since the combination treatment resulted in significantly better effects than the sum of the separate treatments.

## Claims

1. Combination of a PARP inhibitor selected from the group of tetracycline compounds, carboxamido-benzimidazole and 4-hydroxy-quinazoline with an Akt kinase activating compound selected from the group of estrogen compounds and resveratrol for use in the treatment of neurodegenerative disease.

2. The combination for use according to claim 1, where the PARP inhibitor and the Akt kinase activating compound are formulated in the same dosage unit together with auxiliaries generally used in pharmacy.

3. The combination for use according to claim 1, where the PARP inhibitor and the Akt kinase activating compound are formulated in separate dosage units together with auxiliaries generally used in pharmacy, where the dosage units containing the different active agents are placed in a kit and the kit contains instructions for the dosage regimen.

4. The combination for use according to any of claims 1 to 3, wherein the neurodegenerative disease is selected from multiple sclerosis, encephalomyelitis, Parkinson's, Alzheimer's and Huntington's disease.

5. Pharmaceutical composition, which contains a PARP inhibitor selected from the group of tetracycline compounds, carboxamido-benzimidazole and 4-hydroxy-quinazoline in combination with an Akt kinase activating compound selected from the group of estrogen compounds and resveratrol together with auxiliaries generally used in pharmacy.

## Patentansprüche

1. Kombination eines PARP Inhibitors gewählt aus der Gruppe von Tetracyclin-Verbindungen, Karboxamido-benzimidazol und 4-Hydroxy-quinazolin mit einer eine Akt-Kinase aktivierenden Verbindung gewählt aus der Gruppe von Östrogen-Verbindungen und Resveratrol zur Verwendung in der Behandlung von neurodegenerativen Krankheiten.

2. Die Kombination zur Verwendung gemäss Anspruch 1, worin der PARP Inhibitor und die die Akt-Kinase aktivierende Verbindung in derselben Dosiseinheit formuliert sind, zusammen mit in der Pharmazie allgemein verwendeten Hilfsstoffen.

3. Die Kombination zur Verwendung gemäss Anspruch 1, worin der PARP Inhibitor und die die Akt-Kinase aktivierende Verbindung in separaten Dosiseinheiten formuliert sind, zusammen mit in der Pharmazie allgemein verwendeten Hilfsstoffen, worin die die verschiedenen aktiven Stoffe enthaltenden Dosiseinheiten in einer Garnitur angeordnet sind, und die Garnitur Instruktionen für das Dosissystem enthält.

4. Die Kombination zur Verwendung gemäss einem der Patentansprüche 1 bis 3, worin die neurodegenerative Krankheit aus der Gruppe von Sklerosis Multiplex, Encephalomyelitis, Parkinson Krankheit, Alzheimer Krankheit und Huntington Krankheit gewählt ist.

5. Pharmazeutische Komposition, die einen PARP Inhibitor aus der Gruppe von Tetracyclin-Verbindungen, Karboxamido-Benzimidazol und 4-Hydroxy-Quinazolin gewählt ist, in Kombination mit einer die Akt-Kinase aktivierenden Verbindung gewählt aus der Gruppe Östrogen-Verbindungen und Resveratrol, zusammen mit in der Pharmazie allgemein verwendeten Hilfsstoffen.

## Revendications

1. Combinaison d'un inhibiteur PARP choisi dans le groupe de composés de tétracycline, de carboxamido-benzimidazole et de 4-hydroxy-quinazoline avec un composé d'activation de l'act kinase, choisi dans le groupe de composés d'oestrogène et de resvératrol à utiliser dans le traitement de maladies neurodégénératives.

2. La combinaison à utiliser conformément à la revendication 1, dans laquelle l'inhibiteur PARP et le composé d'activation de l'act kinase sont formulés dans la même unité de dosage ensemble avec les auxiliaires généralement utilisés en pharmacie.

3. La combinaison à utiliser conformément à la revendication 1, dans laquelle l'inhibiteur PARP et le composé d'activation de l'act kinase sont formulés dans des unités de dosage séparées avec les auxiliaires généralement utilisés en pharmacie, où les unités de dosage contenant les différents agents actifs sont placées dans un kit contenant des instructions concernant la posologie.

4. La combinaison à utiliser conformément à l'une quelconque des revendications 1 à 3, dans laquelle la maladie neurodégénérative est choisie parmi la sclérose en plaques, l'encéphalomyélite, la maladie de Parkinson, d'Alzheimer et de Huntington.

5. Composition pharmaceutique contenant un inhibiteur PARP choisi dans le groupe de composés de tétracycline, de carboxalido-benzilidazole et de 4-hydroxy-quinazoline en combinaison avec un composé d'activation de l'act kinase choisi dans le groupe de composés d'oestrogène et de resvératrol ensemble avec des auxiliaires généralement utilisés en pharmacie.
